Europäisches Patentamt

(19) European Patent Office

Office européen des brevets

(11) Publication number: **0 100 673**
**A2**

# (12) EUROPEAN PATENT APPLICATION

(21) Application number: **83304437.3**

(22) Date of filing: **01.08.83**

(51) Int. Cl.³: **A 61 K 37/02**

(30) Priority: **02.08.82 US 403934**

(43) Date of publication of application:
**15.02.84 Bulletin 84/7**

(84) Designated Contracting States:
**AT BE CH DE FR GB IT LI LU NL SE –**

(71) Applicant: **THE RESEARCH FOUNDATION OF STATE UNIVERSITY OF NEW YORK**
**PO Box 9**
**Albany New York 12201(US)**

(72) Inventor: **Badalamente, Marie A.**
**109 St. Marks Place**
**Roslyn Heights New York 11577(US)**

(72) Inventor: **Hurst, Lawrence C.**
**17 Caleb-Brewster Rd., E.**
**Setauket New York 11733(US)**

(72) Inventor: **Stracher, Alfred**
**47 The Oaks**
**Roslyn New York(US)**

(74) Representative: **Paget, Hugh Charles Edward et al,**
**MEWBURN ELLIS & CO. 2/3 Cursitor Street**
**London EC4A 1BQ(GB)**

(54) **A method of enhancing neurofiber regrowth.**

(57) A composition for enhancing neurofiber regrowth in mammals contains leupeptin.

Croydon Printing Company Ltd.

# A METHOD OF ENHANCING NEUROFIBER REGROWTH

## BACKGROUND OF THE INVENTION

Nerve repair, or neurorrhaphy, has been done since the 19th century. The use of the operative microscope in such surgery was introduced in 1921 by Nyland. Microsurgical techniques are quite advanced. Despite this, the prognosis for an individual patient is difficult to give accurately. It is well known that many factors, such as the patient's age, the cause of injury, the associated injuries, the extent and type of nerve injury, the location of transsection (high or low), the timing of the repair and the technique of repair all effect the end result after nerve repair (neurorrhaphy). The search for better suture materials, scar preventing techniques and an answer to the epineural versus perineural repair controversy still continues. See, for instance, Terzis, "Neural Microsurgery", Reconstructive Microsurgery, edited by Daniel, and Terzis, Little, Brown and Company, Boston, 1977, pp. 295-478; Bora, "Nerve Response to Injury and Repair," Rehabilitation of the Hand, edited by Hunter, et. al., The C.V. Mosby Company, St. Louis, 1978, pp. 269-272; and Cabaud, et. al., J.P. Hand Surgery 1:131-137, 1976. It is still conceded by most authorities that excellent results after neurorrhaphy still cannot be consistently obtained. The state of art is best summarized by Terzis, "in spite of the recent introduction of higher magnification, finger sutures, smaller instruments and refinement of surgical techniques, the improvement in the overall results of peripheral nerve repairs in the last 100 years as determined by the return of function has been hardly measurable". [Terzis, J. Hand Surgery, 1:52-66, (1976)].

Thus, any adjunctive therapy which could improve the present results would be extremely useful. Our invention comprises an adjunctive therapy which enhances neurofiber regrowth in mammals after neurorrhaphy.

Leupeptin was identified in culture filtrate of actinomycetes by Umezawa and co-workers. [Aoyagi et. al., J. Antibiotics, 22:283-286, (1969); and Kondo, et. al., Chem. Pharm. Bull:, 17:1896-1901, (1969)]. This compound is a mixture of two major constituents, acetyl-L-leu-L-leu-L-argininal and propionyl-L-leu-L-leu-L-argininal. It was further found that either of the L-leu components may be replaced by L-isoleucine (L-isoleu)or L-valine, (L-val). [Kawamure, et. al., Chem. Pharm. Bull., 17:1902-1909, (1969)]. The aldehyde group present at the C-terminal position is considered to be very important for its potent biological inhibitory activity. Leupeptin has been shown to be an effective inhibitor of trypsin, plasmin, kaleikrein, thrombokinase and papain. It has also been established that cathespin B, cathespin L, and calcium activated neutral protease are also inhibited by leupeptin in muscle, squid axoplasm and rat sciatic nerve. Recently, it has been reported that leupeptin was successfully used in vivo as an inhibitor of denervation induced atrophy in chicken pectoralis muscle. [(Stracher et. al., Exp. Neurol., 66: 611-618, (1979)].

Unpublished work by Stracher indicates that the material which was used to carry out these studies was substantially pure (i.e. about 98% by high performance liquid chromatography) acetyl-L-leu-L-leu- argininal. It has been further indicated that the nature of the blocking group at the terminal leucine (or its replacement amino acid) is irrevelant, any naturally occuring or synthetic non-toxic amine blocking group could be used. For example, propionyl or benzoyl groups could be used just as well. This designation not being intended to be critical or limiting.

Furthermore, investigations in chicken and rat skeletal and fetal heart preparations that leupeptin decreased protein degradation while having no effect on protein synthesis. [Libby, et. al., Science, 199 (3): 534-536, (1978); Libby and Goldberg, Am. J. Physiol., 231:441, (1976); and Libby, et. al., Cell, 19:481-491, (1980)]. In addition it has been concluded that leupeptin acts selectively without producing toxic effects or adverse immunogenic reactions. [Umezawa, H. Enzyme Inhibitors of Microbial Origin, University Park Press, Baltimore, 1972].

## SUMMARY OF THE INVENTION

The present invention concerns the enhancement of neurofiber regrowth. More particularly, this invention relates to a method of enhancing neurofiber regrowth in mammals which comprises administering a neurofiber regrowth enhancing amount of leupeptin in a pharmaceutically acceptable vehicle.

## DESCRIPTION OF THE INVENTION

In view of the unclarity of prior nomenclature some clarification in this specification is desirable. Thus, where the term "leupeptin" is used hereinafter and in the claims, it shall be intended to define a compound selected from the group having the formula

$$B-A_1-A_2-L-argininal$$

wherein B is any pharmacologically acceptable amine blocking group suitably acetyl, propionyl or benzoyl but not limited thereto, $A_1$ and $A_2$ are amino acids selected from the group consisting of L-leucine, L-valine and L-isoleucine. The term "leupeptin" when used per se shall also include mixtures of any compounds falling within the above definition.

Where the term "leupeptin" is used with prefixes only, the prefixes shall indicate the nature of the blocking group thus Ac, Pr and Bz shall designate the blocking groups acetyl, propionyl and benzoyl. When used with a prefix and no suffix the term "leupeptin" shall mean L-leu-L-leu-L-argininal. Thus, Ac leupeptin means acetyl-L-leu-L-leu-L-argininal. Similarly, suffixes shall indicate the changes in the first two amino acids thus, leupeptin LV means L-leu-L-Val-L-argininal.

Leupeptin LI means L-leu-L-isoleu-L-argininal.
Leupeptin VI means L-Val-L-isoleu-L-argininal.
Leupeptin VL means L-val-L-leu-L-argininal.
Leupeptin IV means L-isoleu-L-val-L-argininal.
Leupeptin VV means L-val-L-val-L-argininal.
Leupeptin II means L-isoleu-L-isoleu-L-argininal.
Leupeptin IL means L-isoleu-L-leu-L-argininal.

Similarly, the prefix Ac, Pr or Bz indicates the nature of the blocking group. Thus, Ac leupeptin LV means acetyl-L-leu-L-val-L-argininal.

According to the present invention, it has now been found that leupeptin can be utilized to enhance neurofiber regrowth in mammals after microsurgical neurorrhaphy.

While the effectiveness of Ac leupeptin has been demonstrated after surgical severence of neurofibers, its utility is not limited to its use after microsurgical neurorrhaphy since its ability to regenerate neurofibers depends on its enhancement of growth of unmyelinated axonal sprouts. Thus, leupeptin may be used for this purpose in neuro degenerative disorders such as spinal muscular atrophies such as Kugelbeg-Weylander Disease and Werdnig-Hoffman Disease; peripheral neuropathies such as Charcot-Marie Tooth Disease and Peroneal Atrophy, Amyotrophic Lateral Sclerosis, Polio myelites, Guillain-Barre disease, Spinal Trauma, Diabetic Neuropathy, Compression type nerve injuries such as endopathic neurapathy, neuropraxia and traction nerve injuries.

In order to achieve most efficient use of leupeptin it is desirable to provide it to the injured site as rapidly as possible. Hence, for example, in a micro surgical neurorraphy situation it is desirable to initially encourage unmyelinated axonal sprouting as rapidly as possible. Hence, the repair site should be bathed in a leupeptin solution. Since leupeptin is substantially non-toxic the strength of the solution is not critical. A solution of leupeptin or its salts or in a saline solution suitably phosphate buffered saline in generally pharmacological accepted strength, may contain between 5 and 20% by weight preferably between about 10 and 15% by weight of leupeptin.

In non-surgical or post-surgical situations administration may be parenteral or subcutaneous by injection or infusion. Long term administration of leupeptin may suitably be provided by oral administration as a solution, suspension, elixir, tablet, or capsule. When administered by injection the carrier, suitably physiological saline or phosphate buffered saline, may contain between 5 to 20% suitably 10 to 15% by weight of leupeptin or its salts. When administered orally in the form of capsules or tablets said capsules or tablets may contain between about 200 to about 500 milligrams of the active substance per unit of dose, however this formulation should not be considered to be critical but merely a matter of convenience. The total amount administered per day depends upon the opinion of the treating physician but may lie between 400-4,000 milligrams per day for the average human adult.

Leupeptin may be utilized per se or in the form of any of its pharmaceutically acceptable acid derived salts. Among the suitable acids may be mentioned inorganic or organic acids, suitably hydrochloric acid, sulfuric acid, hydrobromic acid, nitric acid, phosphoric acid, formic acid, butyric acid, benzoic acid, nicotinic acid, tartaric acid, glucose-1-phosphoric acid, embonic acid and ethane sulfonic acid. The foregoing acids are recited for purposes of illustration and should no way be considered to be critical or limiting.

Leupeptin is commercially available as the Ac leupeptin hemi sulfate and therefore it would be expected to be utilized in this form or in the form of the hydrochloride or salt.

The enchancement of neurofiber regrowth activity of leupeptin in mammals after microsurgical neurorrhaphy is demonstrated by the results of testing in the following procedures.

Twenty rats are anesthetized with Rompun (0.5 cc total volume). The right sciatic nerve is exposed and severed with microneuroscissors, and subsequently repaired microsurgically with 6-8 circumferential microsutures using 10-0 nylon. Immediately following surgery the treated animals receive 12 mg/kg Ac leupeptin in 5% DMSO (dimethylsulphoxide) as a bath on their sciatic nerve, 0.25 cc. total volume. Then the treated animals are injected intramuscularly in right hindlimb with 0.25 cc of a solution containing 12 mg/kg Ac leupeptin in 5% DMSO. Control animals receive the same surgical treatment, but the bath and intramuscular injection consist only of the solvent and 5% DMSO.

Thereafter, treated animals are injected twice weekly in the operated hindlimb with 12 mg/kg Ac leupeptin in 5% DMSO (0.25 cc total volume) for periods of 1 week, 1 month, two months, three months and six months. Control animals are injected with 0.25 cc of 5% DMSO twice weekly in the operated hindlimb for the same time period.

Two treated and two control animals are assigned to each of the stated time periods.

All animals are sacrified by an overdose of pentobarbital, 1.0 cc total volume. The gastro-cnemius, soleus and extensor digitorum longus muscles of the operated hindlimb are execised from origin to insertion, weighed and then frozen in isopentane in liquid nitrogen at -20°C. For light microscopy, cryostat sections are cut, placed on slides and stained with hematoxylin and eosin and/or ATPase histochemistry at pH's of 4.3 and 10.4. Myofiber diameters are determined by photographing hematoxylin and eosin sections at a constant magnification in the light microscope.

A ten mm. portion of the repaired sciatic nerve is excised from each animal. This is fixed in

7

8

3.0% glutaraldehyde in 0.1 M sodium cacodylate, pH 7.3, overnight for electron microscopy. After post-fixation in 1.0% osmium tetroxide, ethanol dehydration and embedding in Polybed 812, both thick (1 micron) and thin (500 A°) transverse sections are cut on a ultramicrotome. Portions of the excised muscles receive similar preparation for electron microscopy.

Thick sections of the sciatic nerve are placed on microscope slots stained with toluidine blue and photographed at a constant magnification in the light microscope. Thin sections of both nerve and muscles are placed on 300 mesh copper grid calm, stained with uranyl acetate and lead citrate and photographed using a Hitachi HU-12 electron microscope.

The results of the above testing are given in the following tables I-V.

TABLE I

#### Average Wet Weight (g) of Muscles

| | E.D.L. | GASTROCNEMIUS | SOLEUS |
|---|---|---|---|
| One Week Treated | 0.116 | 2.379 | 0.335 |
| One Week Control | 0.096 | 1.519 | 0.292 |
| % Weight (+) gain (−) loss Treated vs. control | +18% | +37% | +13% |
| One Month Treated | 0.198 | .954 | .379 |
| One Month Control | 0.076 | 0.665 | 0.308 |
| % Weight (+) gain or (−) loss Treated vs. control | +62% | +30 | +19 |

(Table 1 continued)

| | | | |
|---|---|---|---|
| Two Months Treated | 0.202 | 1.371 | 0.425 |
| Two Month Control | 0.115 | 1.183 | 0.387 |
| % Weight (+)gain or (-)loss Treated vs. Control | +43% | +14% | +9% |
| Three Month Treated | 0.178 | 1.563 | 0.248 |
| Three Month Control | 0.155 | 1.278 | 0.226 |
| % Weight (+)gain or (-)loss | +13 | +18 | +9 |
| Six Month Treated | 0.163 | 1.577 | 0.306 |
| Six Month Control | 0.134 | 1.355 | 0.235 |
| % Weight (+)gain or (-)loss Treated vs. Control | +18% | +15% | +23% |

In all cases the average wet weight in grams in Ac leupeptin treated muscles is significantly greater than control.

TABLE II

**0100673**

Average Myofiber Diameter (µm) by Light Microscopy

|  | E.D.L. | GASTROCNEMIUS | SOLEUS |
|---|---|---|---|
| One Week Treated | 44.6 | 62.3 | 49.2 |
| One Week Control | 42.2 | 48.1 | 44.9 |
| % Difference Treated vs. Control | +5% | +23% | +9% |
| One Month Treated | 34.1 | 34.5 | 39.4 |
| One Month Control | 31.6 | 34.5 | 30.0 |
| % Difference Treated vs. Control | +7% | +3% | +24% |
| Two Month Treated | 28.9 | 35.4 | 42.3 |
| Two Month Control | 26.0 | 29.2 | 38.3 |
| % Difference Treated vs. Control | +10% | +18% | +10% |
| Three Month Treated | 44.3 | 57.2 | 47.9 |
| Three Month Control | 36.5 | 55.3 | 43.5 |
| % Difference Treated vs. Control | +18 | +3% | +9 |
| Six Month Treated | 75.3 | 73.1 | 79.5 |
| Six Month Control | 54.0 | 54.3 | 55.7 |
| % Difference Treated vs. Control | +29% | +26% | +30% |

10

The average gain in wet weight is reflected in denervated muscles as a mean increase in myofiber diameter in Ac leupeptin treated muscles. The general pattern in both treated and control animals is a decreasing myofiber diameter as denervation atrophy occurs. However, Ac leupeptin treated muscle shows a significantly greater myofiber diameter when compared to control.

<div align="center">

Table III

Counts of Myelinated Axons – 5.0 mm Distal to Repair
by Light Microscopy

$1.0 \times 10^5 \ \mu m^2$ – of Area

</div>

| | |
|---|---|
| One Week Treated | 722 |
| One Week Control | 720 |
| % Difference Treated vs. Control | +1% |
| One Month Treated | 1467 |
| One Month Control | 1438 |
| % Difference Treated vs. Control | +2% |
| Two Month Treated | 1625 |
| Two Month Control | 1436 |
| % Difference Treated vs. Control | +12% |
| Three Month Treated | 2808 |
| Three Month Control | 2800 |
| % Difference Treated vs. Control | +1% |
| Six Month Treated | 1620 |
| Six Month Control | 1620 |
| % Difference Treated vs. Control | 0% |

<div align="center">

11

</div>

As Wallerian degeneration occurs proximal to the repair site in sciatic nerve, attempts at regeneration by distal proximal axonal sprouting occurs. This process involves the remyelination of such sprouts. The results indicate that distally, leupeptin treated nerves do not undergo as severe a form of Wallerian degeneration as the control nerve and that regeneration or sprouting from the proximal side is enhanced by leupeptin.

TABLE IV

Counts of Myelinated Axons - 5.0mm. Proximal to Repair
by Light Microscopy
$1.0 \times 10^5 \ \mu m^2$ - of Area

| | |
|---|---|
| One Week Treated | 1089 |
| One Week Control | 911 |
| % Difference Treated vs. Control | +16% |
| One Month Treated | 1162 |
| One Month Control | 1064 |
| % Difference Treated Vs. Control | +7% |
| Two Month Treated | 1111 |
| Two Month Control | 1017 |
| % Difference | +8% |

(Table IV continued)

| | |
|---|---|
| Three Month Treated | 2200 |
| Three Month Control | 1179 |
| % Difference | +46 |

| | |
|---|---|
| Six Month Treated | 1748 |
| Six Month Control | 1218 |
| % Difference Treated vs. Control | +30% |

Counts performed as an indicator of proximal chromatolysis after sciatic severing and repair suggests that such chromatolytic activity is inhibited by leupeptin. In all cases, treated nerves show a significantly higher number of myelinated axons when compared to control.

TABLE V

Counts of Myelinated and Unmyelinated Axons
5.0mm. Distal to Repair - by Electron Microscopy
Per 200 $\mu$m$^2$ of Area

| | #Myelinated Axons | #Unmyelinated Axons |
|---|---|---|
| One week treated | 4.25 | 11.0 |
| One week control | 2.0 | .0 |
| % Difference Treated vs. Control | 53% | >100 |
| One month treated | 1.2 | 8 |
| One month control | 1.6 | 2.4 |
| % Difference Treated vs. Control | 25% | 70% |

(Table V continued)

|  | | |
|---|---|---|
| Two Month Treated | 6 | 18 |
| Two Month Control | 6 | 9 |
| %Difference Treated vs. Control | 0% | 50% |
| Three month treated | 9.0 | 12.9 |
| Three month control | 3.8 | 5 |
| %Difference Treated vs. Control | 58% | 61% |
| Six Month Treated | 5 | 8.7 |
| Six Month Control | 4.5 | 6.0 |
| %Difference Treated vs. Control | 1% | +30 |

Ultrastructural data distal to the repair site indicates increased numbers of both myelinated and unmyelinated axons up to and including 6 months of leupeptin treatment. This indicates that leupeptin inhibits Wallerian degeneration and accelerates the regeneration process as evidenced especially by the increased number of unmyelinated axonal sprouts at a site far distal from the microsurgical repair. .

In summary, the test results shown in tables I-V indicate that leupeptin significantly enhances neurofiber regrowth in mammals after microsurgical neurorrhaphy.

In view of its potent pharmacological property, leupeptin can be combined with pharmaceutical carriers and administered in a variety of well-known pharmaceutical forms suitable for parenteral or oral administration to provide a composition useful in the enhancement of neurofiber regrowth in mammals.

Based upon laboratory tests, the effective dose $ED_{50}$ of leupeptin will typically be in the range of about 8 to about 75 mg/kg, and preferably about 10-50 mg/kg, of mammalian weight administered in single or divided doses. The exact dose to be administered is dependent upon the age, weight and particular conditions of the individual involved.

The compositions containing the compounds of this invention will preferably contain from about 12 mg/kg of the leupeptin per dosage unit. Said compositions may be administered orally but are most preferably administered parenterally near the affected area.

Typical formulations are those such as solutions, suspensions, elixirs, syrups, tablets, or capsules.

Typical acceptable pharmaceutical carriers for use in formulations described above are exemplified by: sugars, such as lactose, sucrose, manitol, and sorbitol; starches such as corn starch, tapioca starch and potato starch; cellulose and derivatives such as sodium carboxymethyl cellulose, ethylcellulose and methylcellulose; polyvinylpyrrolidone; polyvinyl alcohol; stearic acid,vegetable oils such as peanut oil, cottonseed oil, sesame oil, olive oil and corn oil; non-ionic cationic and anionic surfactants; ethylene glycol polymers; water; saline; as well as other non-toxic compatible fillers, binders, disintegrants and lubricants commonly used in pharmaceutical formulations.

It will be apparent to those skilled in the art that many modifications, both of materials and methods, may be practiced without departing from the spirit and scope of the invention.

## EXAMPLE 1

Injectable Formulation

| | |
|---|---|
| Ac Leupeptin | 100 mg. |
| Water for injection (U.S.P) q.s. 2.0 ml | |

In the above formulation Ac leupeptin may be replaced by Ac-leupeptin (LV, LI, VI, VV, IV, II, IL or VL) or their analogs wherein the acetyl group is replaced by propionyl, benzoyl, or other pharmaceutically acceptable amino blocking groups.

## EXAMPLE 2

Injectable Formulation - every 8 hours (3x daily) Ac Leupeptin                    12mg/kg body weight Saline (physiological)
0.9%, Injectable                2.0 ml

In the above formulation Ac leupeptin may be replaced by Ac leupeptin (LV, LI, VI, VV, IV, II, IL or VL) or their analogs wherein the acetyl group is replaced by propionyl, benzoyl or other pharmaceutically acceptable amino blocking groups.

## EXAMPLE 3

PO Formulation   - every 8 hours (3x daily) Ac Leupeptin           12 mg/kg body weight Dissolved in 6 oz. fruit juice

In the above formulation Ac leupeptin may be replaced by Ac leupeptin (LV, LI, VI, VV, IV, II, IL or VL) or their analogs wherein the acetyl group is replaced by propionyl, benzoyl or other pharmaceutically acceptable amino blocking group.

A composition embodying the invention may contain a single, substantially pure leupeptin.

CLAIMS:-

1. A neurofiber growth enhancing composition comprising leupeptin and a pharmacologically acceptable carrier.

2. A composition of claim 1, wherein the leupeptin is Ac leupeptin.

3. A composition of claim 1, wherein the leupeptin is (Ac, Pr or Bz) leupeptin (LV, LI, VI, VV, IV, II, IL or VL) or (Pr or Bz) leupeptin.

4. A composition according to claim 1, claim 2 or claim 3, wherein the leupeptin is a single compound in substantially pure form.

5. A composition of any one of claims 1 to 4, which contains at least about 12 mg/kg of the leupeptin per dosage unit.

6. A method of enhancing neurofiber regrowth in mammals which comprises administering to a mammal in need of treatment, a neurofiber regrowth enhancing amount of leupeptin or any of its pharmaceutically acceptable salts in a pharmaceutically acceptable vehicle.

7. A method of claim 6, wherein leupeptin is (Ac, Pr or Bz) leupeptin (LV, LI, VI, VV, IV, II, IL or VL) or (Pr or Bz) leupeptin.

8. A method according to claim 6, wherein the amount administered is from about 8 to 75 mg/kg/day mammalian weight.

9. A method according to claim 6 or 8 wherein the route of administration is parenteral.

10. A method according to claim 6 or 8 wherein the leupeptin is administered at a site proximate to the microsurgical neurorrhaphy.